# FASCICULE DE BREVET EUROPEEN

(11) **EP 1 247 521 B1**
(45) Date de publication et mention de la délivrance du brevet: **17.11.2004**
(21) Numéro de dépôt: 02290659.8
(22) Date de dépôt: 15.03.2002
(51) Int. Cl.: A61K 7/48, A61K 31/505, A61K 31/155

(54) **Utilisation d'un dérivé de biguanide et d'une pyrimidine pour la fabrication d'une composition topique pour le soin de la peau**
Verwendung von Biguanid und Pyrimidin-Derivaten zur Herstellung topischer Hautpflegezubereitungen
Use of biguanide and pyrimidine derivatives for the preparation of a topical skin care composition

(30) Priorité: 15.03.2001 FR 0103510
(43) Date de publication de la demande: 09.10.2002
(73) Titulaire: Salkin, André, 76302 Sotteville les Rouen Cédex (FR)
(72) Inventeur: Salkin, André, 76302 Sotteville les Rouen Cédex (FR)
(74) Mandataire: Portal, Gérard

(56) Documents cités:
- EP-A- 0 136 231
- EP-A- 0 308 210
- WO-A-95/04520
- GB-A- 2 271 719
- US-A- 4 900 721
- US-A- 5 114 943
- US-A- 5 629 006
- US-A- 5 894 019

## Description

La présente invention concerne l'utilisation d'au moins un dérivé de biguanide et d'au moins une pyrimidine pour la fabrication d'une composition topique pour le soin de la peau, notamment des peaux jeunes ou des peaux à tendance acnéique.

Cependant certaines législations nationales ont des dispositions légales particulières, certains pays considèrent que ces compositions topiques pour le soin de la peau sont des préparations cosmétiques tandis que d'autres pays ont des législations qui considère que ces compositions topiques pour le soin de la peau rentrent dans le cadre de soin pharmaceutique et certains pays n'auront aucune considération particulière. De ce fait, l'invention peut être soumise à cette législation sans que cela implique que cette composition soit considérée comme une composition cosmétique ou pharmaceutique au sens classique ou du droit des brevets. De préférence, le dérivé de biguanide est choisi parmi la chlorhexidine (hexaméthylène-bis-5-(p-chlorophényl)-biguanide) et le PHMB (polyhexaméthylène-biguanide) et la pyrimidine est l'hexetidine (1,3-bis-(β-éthylhexyl)-5-aminohexahydro-pyrimidine).

### ETAT DE LA TECHNIQUE

Les problèmes des peaux abîmées des adolescents sont dus à des germes microbiens présents sur la peau, et causant des lésions inflammatoires (papules, pustules) et des lésions rétentionnelles (kystes, comédons).

Afin de démontrer l'efficacité des produits mis sur le marché pour traiter les problèmes des peaux jeunes, il est usuel d'effectuer des dosages de CMI (Concentration Minimale Inhibitrice) sur 3 souches microbiennes caractéristiques de l'acné :
- *Propionibacrérium acnès*
- *Staphylococcus épidermidis*
- *Corynebacterium xerosis*

La solution classique lors de la mise au point d'une formulation visant à améliorer les problèmes de peaux précités revient à utiliser des molécules bactéricides dosées de telle sorte que les seuils de CMI de ces souches caractéristiques soient atteints.

Il est connu par le brevet d'invention n° EP 0 136 231, que certaines associations d'un biguanide et d'une pyrimidine présentent un pouvoir bactéricide très élevé. Ces compositions peuvent être utilisées dans la désinfection de la peau, par exemple pour le lavage des mains du personnel hospitalier.

Dans le cadre de ce brevet EP 0 136 231, où l'on vise une destruction totale des germes, le dosage des produits actifs est effectué de façon à ce que l'on atteigne au minimum la CMI sur les souches microbiennes que l'on cherche à détruire.

Il est connu que les chlorhexidines sont faiblement irritatives sur les peaux saines, mais il ne serait pas envisageable d'utiliser des doses bactéricides donc importantes de ces molécules sur des zones de peaux fragilisées comme les peaux acnéiques.

Les réactions d'intolérance sont fréquentes, telles que : Irritations des zones sèches du visage (pommettes, tour de l'oeil), brûlures en plaques, desquamation de la peau.

Le caractère irritant de l'hexetidine est également connu de l'homme de l'art, et l'utilisation de cette molécule aux doses permettant d'atteindre les CMI des souches caractéristiques de l'acné serait beaucoup trop irritante pour une utilisation sur des zones de peau fragiles.

### BUTS DE L'INVENTION

La présente invention a pour but principal de résoudre le nouveau problème technique consistant en la fourniture d'une solution permettant de réaliser le soin de la peau notamment des peaux jeunes ou des peaux à tendance acnéique d'une manière simple, efficace à une concentration non bactéricide et sans effet secondaire sensible sur de telles peaux, notamment sans phénomène d'irritation.

La présente invention a encore pour but principal de résoudre le nouveau problème technique consistant en la fourniture d'une solution permettant de réaliser le soin alternatif au traitement habituel ou connu des peaux jeunes abîmées, en particulier des adolescents, ou des peaux acnéiques, ou ayant des lésions inflammatoires telles que papules, pustules, ou des lésions rétentionnelles telles que kystes ou comédons, d'une manière simple, topiquement efficace à une concentration non bactéricide et sans effet secondaire sensible sur lesdites peaux.

L'invention a encore pour but principal de résoudre les deux nouveaux problèmes techniques énoncés ci-dessus de manière simultanée, par la même solution technique d'une manière sûre et fiable à une concentration non bactéricide, utilisable à l'échelle industrielle, et assurant un soin alternatif au traitement habituel des peaux jeunes abîmées.

D'une façon tout à fait surprenante, il a été découvert que des formulations d'associations d'au moins un dérivé de biguanide et d'au moins une pyrimidine, dosées en dessous des CMI permettaient une nette amélioration des problèmes de peau de jeunes sujets volontaires, permettant de réaliser un soin de la peau, pour les sujets ayant auparavant testé des multitraitements.

Ainsi, selon un premier aspect, la présente invention concerne l'utilisation d'au moins un dérivé de biguanide et d'au moins une pyrimidine pour la fabrication d'une composition topique de soin de la peau, notamment des peaux jeunes ou des peaux à tendance acnéique, comprenant au moins un dérivé de biguanide et au moins une pyrimidine, caractérisée en ce que ladite compositon a une activité inférieure à la concentration minimale inhibitrice ou CMI obtenue avec au moins une souche caractéristique de l'acné, la souche propionibacterium acnès ; le staphylococcus épidermidis et le corynebacterium xerosis.

Selon une autre variante, il est réalisé la fabrication d'une composition de soin pour la peau, en soin alternatif au traitement habituel pour le soin topique des peaux jeunes abîmées, en particulier des adolescents, ou pour le soin des peaux acnéiques ou ayant des lésions inflammatoires telles que papules, pustules ou des lésions rétentionnelle telles que kystes ou comédons.

Avantageusement, le dérivé de biguanide est choisi parmi la chlorexhidine et le PHMB, et la pyrimidine est l'hexetidine.

Selon encore une autre caractéristique avantageuse de l'invention, la concentration maximum en dérivé de biguanide est de 1 % en solution à 20 % en poids dans un excipient compatible avec la peau; tandis que la concentration maximum en pyrimidine est de 0,14 % en poids dans un excipient compatible avec la peau.

Les pourcentages pour l'hexetidine peuvent varier en dessous de 0,14 % jusqu'à une valeur encore efficace par exemple de 0,05 %, et pour la chlorexhidine ou le chlorhydrate de PHMB en dessous de 1 % de manière à toujours obtenir une concentration efficace, par exemple 0,5 %.

Selon une réalisation avantageuse la composition comprend 1 % en poids de digluconate de chlorexhidine ou de chlorhydrate de PHMB à 20 % en poids dans un excipient compatible avec la peau et 0,05 % en poids d'hexetidine dans un excipient compatible avec la peau.

Selon un deuxième aspect, la présente invention couvre aussi une composition topique de soin pour la peau, notamment pour le soin des peaux jeunes ou des peaux à tendance acnéique caractérisée en ce qu'elle comprenant au moins un dérivé de biguanide et au moins une pyrimidine caractérisée en ce que la composition a une activité inférieure à la concentration minimum d'inhibition ou CMI d'au moins une souche caractéristique de l'acné, ladite souche caractéristique de l'acné est choisie parmi les souches microbiennes *propionibacterium acnès, staphylococeus epidermis et corynebacterium xerosis*, dans un excipient compatible avec la peau.
Selon un troisième aspect, la présente invention couvre encore une composition topique de soin pour la peau en solution alternative à un traitement habituel pour le soin des peaux abîmées, en particulier des adolescents, ou des peaux acnéiques ou ayant des lésions inflammatoires cutanées, telles que papules, pustules ou de lésions cutanées rétentionnelles telles que kystes ou comédons, caractérisée en ce qu'elle comprend au moins un dérivé de biguanide et au moins une pyrimidine à une concentration combinée inférieure à la concentration minimale inhibitrice ou CMI d'au moins une souche caractéristique de l'acné, ladite souche caractéristique de l'acné est choisie parmi les souches microbiennes *propionibacterium acnès, staphylococeus epidermis et corynebacterium xerosis*, dans un excipient compatible avec la peau.

Dans le cadre de l'un ou l'autre des aspects précédents, la composition est caractérisée de préférence en ce qu'elle comprend 1 % en poids d'au moins un dérivé de biguanide précité à une concentration à 20 % en poids dans un excipient compatible avec la peau ; et 0,05 % en poids d'au moins une pyrimidine dans un excipient compatible avec la peau .

Selon une autre caractéristique avantageuse et préférée de l'invention, la composition précitée comprend 1 % en poids de digluconate de chlorexhidine ou de chlorhydrate de PHMB à 20 % en poids dans un excipient compatible avec la peau ; et de 0,05 % en poids d'hexetidine dans un excipient compatible avec la peau.

La composition précitée peut aussi comprendre au moins un excipient compatible avec la peau et compatible avec les ingrédients actifs de l'invention et adapté à un usage topique sur la peau.

Selon un quatrième aspect, la présente invention couvre aussi un procédé de préparation d'une composition de traitement ou de soin topique de la peau, notamment d'une peau jeune ou d'une peau à tendance acnéique, ou de traitement des peaux abîmées, en particulier des adolescents, ou des peaux acnéiques ou ayant des lésions inflammatoires, telles que papules, pustules, ou des lésions rétentionnelles telles que kystes ou comédons, caractérisé en ce qu'on prépare la composition telle que précédemment définie.

Des variantes de réalisation avantageuses de ces procédés résultent clairement de la description précédente relativement aux autres aspects de l'invention, ainsi que de la description suivante.

D'autres buts, caractéristiques et avantages de l'invention apparaîtront clairement à partir de la description explicative qui va suivre faite en référence à plusieurs exemples de réalisation de l'invention donnés simplement à titre d'illustration et qui ne sauraient donc en aucune façon limiter la portée de l'invention. Les exemples font partie intégrante de l'invention. En outre, dans la totalité de la description et des revendications, tous les pourcentages sont donnés en poids sauf indication contraire. La température est la température ambiante et la pression est la pression atmosphérique.

### EXEMPLE 1 de l'invention

A - On prépare une composition N°1 selon l'invention suivante :

### Composition N°1 selon l'invention :

| Principe actif | Concentration utilisée (% w/w) |
|---|---|
| Digluconate de chlorhexidine dilué à 20 % | 1 % |
| Hexetidine | 0.05 % |

B - On réalise un test de concentration minimum inhibitrice relativement à trois souches caractéristiques de l'acné, de la manière suivante, avec la composition N°1 comprenant pour principaux actifs une association de digluconate de chlorhexidine et d'hexetidine en faibles proportions.

### Test de CMI sur la composition N° 1 selon l'invention :

**TABLEAU I**

| Souche | Contamination de départ (germes / g) | Contamination après 48 heures | | |
|---|---|---|---|---|
| | | Composition pure | Composition diluée à 50 % | Composition diluée à 25 % |
| *Propionibacterium acnès* | 4,2. 10⁷ | 5. 10⁶ | 13. 10⁶ | 18. 10⁶ |
| *Staphylococcus épidermidis* | 5,5. 10⁶ | 25.10¹ | 23. 10² | 4. 10³ |
| *Corynebacterium xerosis* | 3. 10⁶ | 9. 10¹ | 7. 10³ | 8. 10⁴ |

Conclusion du test : Les doses mises en oeuvre dans la composition N° 1 selon l'invention ne permettent pas d'atteindre la CMI.

### C - Etude de la composition N° 1 selon l'invention :

On réalise une étude de la manière suivante :
Nombre de sujets : 36
Age : 16 à 25 ans
Critères d'inclusion : lésions inflammatoires et rétentionnelles importantes sur le visage.

### Résultats du test :

**TABLEAU II**

| Composition | Type de lésions | Nombre de lésions | J0 | J28 | Différence | Probabilité (test unilatéral) |
|---|---|---|---|---|---|---|
| N° 1 36 sujets | Inflammatoires | Moyenne | 16.56 | 10.31 | -37 % | p = 0.0005 |
| | | Ecart Type | 15.84 | 15.27 | | |
| | Rétentionnelles | Moyenne | 29.17 | 21.86 | -23 % | p = 0.02 |
| | | Ecart Type | 32.60 | 25.38 | | |

La probabilité calculée selon un test unilatéral de Wilcoxon pour ces tests indique une significativité des résultats pour p < 0.05.

Il apparaît à la lecture de ces résultats que la composition testée permet de diminuer de façon significative le nombre de lésions des sujets. En effet, les lésions inflammatoires ont été réduites de 37 % avec p = 0.0005 (hautement significatif), et les lésions rétentionnelles de 23 % avec p = 0.02 (très significatif).

Aucune réaction d'intolérance n'a été détectée pendant l'étude.

### Essai comparatif entre la composition N°4 selon l'invention et diverses compositions comparatives respectivement N°2, N°3 et N°5

Au vu des résultats de l'exemple 1, plusieurs compositions ont été formulées, dosées faiblement, de telle sorte que les molécules mises en oeuvre ne soient pas bactéricides (doses inférieures aux CMI) et à faible caractère irritant, à savoir les compositions du tableau III, dont on observera que les compositions N°2, N°3 et N°5 sont comparatives, tandis que la composition N°4 est une composition selon l'invention comprenant une association critique d'hexetidine et de chlorhydrate de PHMB diluée à 20 % dans de l'eau déminéralisée.

**TABLEAU III**

| | Principe actif | Concentration utilisée (% w/w) |
|---|---|---|
| Composition 2 comparative | Digluconate de chlorhexidine dilué à 20 % | 0% |
| | Hexetidine | 0.05 % |
| Composition 3 comparative | Digluconate de chlorhexidine dilué à 20 % Hexetidine | 1 % |
| | | 0 % |
| Composition 4 selon l'invention | Chlorhydrate de PHMB dilué à 20 % Hexetidine | 1 % |
| | | 0.05 % |
| Composition 5 comparative | Chlorhydrate de PHMB dilué à 20 % Hexetidine | 1 % |
| | | 0 % |

On observe du tableau III qu'aucune de ces compositions n'a permis d'atteindre les CMI sur les 3 souches caractéristiques.

Les tests ont été effectués dans les mêmes conditions que pour la composition N° 1 selon l'invention, et fait l'objet du tableau IV.

Aucune réaction d'intolérance n'a été soulevée durant ces tests.

En revanche, comme le montre le tableau IV, les compositions comparatives qui ne contenaient qu'un actif (N° 2,3 et 5) n'ont pas fourni de résultats probants : La réduction des lésions n'a pas dépassé 8%, avec des probabilités indiquant des résultats non significatifs.

**TABLEAU IV**

| Composition | Type de lésions | Nombre de lésions | J0 | J28 | Différence | Probabilité (test unilatéral) |
|---|---|---|---|---|---|---|
| N° 2 33 sujets | Inflammatoires | Moyenne | 17.36 | 16.12 | -7 % | p = 0.096 N.S. |
| | | Ecart Type | 17.87 | 17.28 | | |
| | Rétentionnelles | Moyenne | 23.7 | 22.15 | - 6 % | p = 0.151 N.S. |
| | | Ecart Type | 21.56 | 22.40 | | |
| N° 3 39 sujets | Inflammatoires | Moyenne | 24.13 | 22.13 | - 8 % | p = 0.072 N.S. |
| | | Ecart Type | 23.43 | 22.75 | | |
| | Rétentionnelles | Moyenne | 33.10 | 31.38 | - 5 % | p = 0.221 N.S. |
| | | Ecart Type | 27.15 | 25.75 | | |
| N° 4 41 sujets | Inflammatoires | Moyenne | 16.68 | 9.80 | -41 % | p = 0.000 Hautement significatif |
| | | Ecart Type | 17.03 | 13.53 | | |
| | Rétentionnelles | Moyenne | 28.22 | 22.12 | - 22 % | p = 0.020 Très significatif |
| | | Ecart Type | 27.81 | 18.55 | | |
| N° 5 40 sujets | Inflammatoires | Moyenne | 15.15 | 15.40 | + 2 % | p = 0.293 N.S. |
| | | Ecart Type | 16.21 | 15.79 | | |
| | Rétentionnelles | Moyenne | 30.88 | 28.57 | -7 % | p = 0.248 N.S. |
| | | Ecart Type | 29.82 | 27.72 | | |

| | | | | | | |
|---|---|---|---|---|---|---|
| N.S. : Non Significatif | | | | | | |

Seule la composition N° 4 selon l'invention, qui contenait 2 molécules (hexétidine et PHMB) a démontré une activité similaire à celle de la composition N°1 selon l'invention : réduction de 41 % des lésions inflammatoires (p = 0.000 : hautement significatif) et réduction de 22% des lésions rétentionnelles (p = 0.02 : très significatif).

Dans le cadre de la présente invention il apparaît donc que l'association non bactéricide d'hexetidine avec un dérivé de biguanide tel que la chlorhexidine ou le PHMB présente une activité de soin des peaux jeunes acnéiques ou des peaux à tendance acnéiques démontrée par d'excellents résultats qui constituent une méthode sûre et fiable pour prouver l'efficacité du soin.

## Revendications

1. Utilisation d'au moins un dérivé de biguanide et d'au moins une pyrimidine pour la fabrication d'une composition topique de soin de la peau, notamment des peaux jeunes ou des peaux à tendance acnéique comprenant au moins un dérivé de biguanide et au moins une pyrimidine, **caractérisée en ce que** ladite composition a une activité inférieure à la concentration minimale inhibitrice ou CMI obtenue avec au moins une souche caractéristique de l'acné, choisie parmi les souches microbiennes *propionibacterium acnès, staphylococcus epidermidis et corynebacterium xerosis*.

2. Utilisation selon la revendication 1, **caractérisée en ce que** le dérivé de biguanide est choisi parmi la chlorexhidine et le PHMB, et la pyrimidine est l'hexetidine.

3. Utilisation selon l'une des revendications précédentes, **caractérisée en ce que** la concentration maximale en dérivé de biguanide est de 1 % en solution à 20 % en poids dans un excipient acceptable, compatible avec la peau et la concentration maximale en pyrimidine est de 0,14 %, la concentration étant de préférence 0,05 % en poids dans un excipient acceptable, compatible avec la peau.

4. Utilisation selon l'une des revendications précédentes, **caractérisée en ce que** la composition comprend 1 % en poids de digluconate de chlorexhidine ou de chlorhydrate de PHMB à 20 % en poids dans un excipient acceptable, compatible avec la peau et 0,05 % en poids d'hexetidine dans un excipient acceptable, compatible avec la peau.

5. Composition topique pour le soin de la peau comprenant au moins un dérivé de biguanide et au moins une pyrimidine, **caractérisée en ce que** la composition a une activité inférieure à la concentration minimum d'inhibition ou CMI d'au moins une souche caractéristique de l'acné, ladite souche caractéristique de l'acné est choisie parmi les souches microbiennes *propionibacterium acnès, staphylococcus epidermidis et corynebacterium xerosis*, dans un excipient compatible avec la peau.

6. Composition topique selon la revendication 5, pour le traitement des peaux abîmées, en particulier des adolescents, ou des peaux acnéiques ou ayant des lésions inflammatoires cutanées, telles que papules, pustules ou de lésions cutanées rétentionnelles telles que kystes ou comédons, **caractérisée en ce qu'**elle comprend au moins un dérivé de biguanide et au moins une pyrimidine et ladite composition a une activité inférieure à la concentration minimum inhibitrice ou CMI d'au moins une souche caractéristique de l'acné, ladite souche caractéristique de l'acné est choisie parmi les souches microbiennes *propionibacterium acnès, staphylococcus epidermidis et corynebacterium xerosis*, dans un excipient compatible avec la peau.

7. Composition selon la revendication 5 ou 6, **caractérisée en ce qu'**elle comprend 1 % en poids d'au moins un dérivé de biguanide précité à une concentration à 20 % en poids dans un excipient acceptable, compatible avec la peau, et une concentration maximale de 0,14 %, la concentration étant de préférence 0,05 % en poids, d'au moins une pyrimidine.

8. Composition selon l'une des revendications 5 à 7, **caractérisée en ce qu'**elle comprend 1 % en poids de digluconate de chlorexhidine ou de chlorhydrate de PHMB à 20 % en poids dans un excipient acceptable, compatible avec la peau, et 0,05 % en poids d'hexetidine.

9. Procédé de préparation d'une composition de traitement ou de soin topique de la peau, notamment d'une peau jeune ou d'une peau à tendance acnéique, ou de traitement des peaux abîmées, en particulier des adolescents, ou des peaux acnéiques ou ayant des lésions inflammatoires cutanées, telles que papules, pustules ou de lésions cutanées rétentionnelles telles que kystes ou comédons, **caractérisé en ce qu'**on prépare la composition telle que définie à l'une quelconque des revendications 5 à 8.

## Patentansprüche

1. Verwendung von mindestens einem Biguanidderivat und mindestens einem Pyrimidin zur Herstellung einer topischen Hautpflegezubereitung, insbesondere für junge Haut oder Haut, die zu Akne neigt, umfassend mindestens ein Biguanidderivat und mindestens ein Pyrimidin, **dadurch gekennzeichnet, daß** die Zubereitung eine Aktivität unter der minimalen Hemmkonzentration oder MHK aufweist, die mit mindestens einem charakteristischen Aknestamm, ausgewählt unter den Mikrobenstämmen *Propionibacterium acnes, Staphylococcus epidermis* und *Corynebacterium xerosis,* erhalten wird.

2. Verwendung nach Anspruch 1, **dadurch gekennzeichnet, daß** das Biguanidderivat unter Chlorhexidin und PHMB ausgewählt ist und das Pyrimidin Hexetidin ist.

3. Verwendung nach einem der vorhergehenden Ansprüche, **dadurch gekennzeichnet, daß** die maximale Konzentration Biguanidderivat 1 % in Lösung bis 20 Gew.-% in einer geeigneten hautverträglichen Grundmasse beträgt und die maximale Pyrimidinkonzentration 0,14 % beträgt, wobei die Konzentration vorzugsweise 0,05 Gew.-% in einer geeigneten hautverträglichen Grundmasse beträgt.

4. Verwendung nach einem der vorhergehenden Ansprüche, **dadurch gekennzeichnet, daß** die Zubereitung 1 Gew.-% Chlorhexidindiglukonat oder PHMB-Chlorhydrat bis 20 Gew.-% in einer geeigneten hautverträglichen Grundmasse und 0,05 Gew.-% Hexetidin in einer geeigneten hautverträglichen Grundmasse umfaßt.

5. Topische Hautpflegezubereitung, die mindestens ein Biguanidderivat und mindestens ein Pyrimidin umfaßt, **dadurch gekennzeichnet, daß** die Zubereitung eine Aktivität unter der minimalen Hemmkonzentration oder MHK von mindestens einem charakteristischen Aknestamm aufweist, wobei der charakteristische Aknestamm unter den Mikrobenstämmen *Propionibacterium acnes, Staphylococcus epidermis* und *Corynebacterium xerosis* in einer hautverträglichen Grundmasse ausgewählt ist.

6. Topische Zubereitung nach Anspruch 5 zur Behandlung von Hautschäden, insbesondere bei Jugendlichen, oder von Aknehaut oder Haut mit entzündlichen Hautläsionen wie z.B. Papula, Pusteln oder Hautretentionsläsionen wie z.B. Zysten oder Mitesser, **dadurch gekennzeichnet, daß** sie mindestens ein Biguanidderivat und mindestens ein Pyrimidin umfaßt, wobei die Zubereitung eine Aktivität unter der minimalen Hemmkonzentration oder MHK von mindestens einem charakteristischen Aknestamm aufweist, wobei der charakteristische Aknestamm unter den Mikrobenstämmen *Propionibacterium acnes, Staphylococcus epidermis* und *Corynebacterium xerosis* in einer hautverträglichen Grundmasse ausgewählt ist.

7. Zubereitung nach Anspruch 5 oder 6, **dadurch gekennzeichnet, daß** sie 1 Gew.-% von mindestens einem vorgenannten Biguanid in einer Konzentration bis 20 Gew.-% in einer geeigneten hautverträglichen Grundmasse und eine maximale Konzentration von 0,14 Gew.-%, vorzugsweise 0,05 Gew.-% von mindestens einem Pyrimidin umfaßt.

8. Zubereitung nach einem der Ansprüche 5 bis 7, **dadurch gekennzeichnet, daß** sie 1 Gew.-% Chlorhexidindiglukonat oder PHMB-Chlorhydrat bis 20 Gew.-% in einer geeigneten hautverträglichen Grundmasse und 0,05 Gew.-% Hexetidin umfaßt.

9. Verfahren zur Herstellung einer topischen Hautbehandlungs- oder -pflegezubereitung, insbesondere für junge Haut oder Haut, die zu Akne neigt, oder zur Behandlung von Hautschäden, insbesondere bei Jugendlichen, oder von Aknehaut oder Haut mit entzündlichen Hautläsionen wie z.B. Papula, Pusteln oder Hautretentionsläsionen wie z.B. Zysten oder Mitessern, **dadurch gekennzeichnet, daß** die Zubereitung hergestellt wird wie es in einem beliebigen der Ansprüche 5 bis 8 definiert ist.

## Claims

1. Use of at least one biguanide derivative and of at least one pyrimidine to prepare a topical skin care composition, in particular for young or acne-prone skins, comprising at least one biguanide derivative and at least one pyrimidine, **characterized in that** the activity of said composition is lower than the minimum inhibitor concentration or MIC obtained with at least one strain characteristic of acne, chosen from among the microbial strains *propionibacterium acnes, staphylococcus epidermis* and *corynebacterium xerosis*.

2. Use as in claim 1, **characterized in that** the biguanide derivative is chosen from among chlorhexidine and PHMB, and the pyrimidine is hexetidine.

3. Use as in any of the preceding claims, **characterized in that** the maximum concentration of biguanide derivative is 1% in solution at 20% by weight in a suitable skin-compatible excipient, and the maximum concentration of pyrimidine is 0.14%, the concentration preferably being 0.05% by weight, in a suitable excipient compatible with the skin.

4. Use as in any of the preceding claims, **characterized in that** the composition comprises 1 % by weight chlorhexidine digluconate or PHMB hydrochlorate at 20 % by weight in a suitable excipient compatible with the skin, and 0.05 % by weight hexetidine in a suitable excipient compatible with the skin.

5. Topical skin care composition comprising at least one biguanide derivative and at least one pyrimidine, **characterized in that** the activity of the composition is less than the minimum inhibitor concentration or MIC of at least one strain characteristic of acne, said strain characteristic of acne being chosen from among the microbial strains *propionibacterium acnes, staphylococcus epidermis* and *corynebacterium xerosis,* in a skin-compatible excipient.

6. Topical composition as in claim 5 to treat damaged skin, in particular in young persons, or acne-prone skins or having inflammatory skin lesions such as papules, pustules, or retention skin lesions such as cysts or comedones, **characterized in that** it comprises at least one biguanide derivative and at least one pyrimidine, and the activity of said composition is lower than the minimum inhibitor concentration MIC of at least one strain characteristic of acne, said strain characteristic of acne being chosen from among the microbial strains *propionibacterium acnes, staphylococcus epidermis* and *corynebacterium xerosis* in an excipient compatible with the skin.

7. Composition as in claim 5 or 6, **characterized in that** it comprises 1 % by weight of at least one above-cited biguanide derivative at a concentration of 20 % by weight in a suitable excipient compatible with the skin, and a maximum concentration of 0.14 %, the concentration preferably being 0.05 % by weight, of at least one pyrimidine.

8. Composition as in any of claims 5 to 7, **characterized in that** it comprises 1 % by weight of chlorhexidine digluconate or PHMB hydrochlorate at 20 % by weight in a suitable excipient compatible with the skin, and 0.05% by weight hexetidine.

9. Method for preparing a topical skin care composition, in particular for young or acne-prone skins, or for treating damaged skin in particular in young persons, or acne skins or inflammatory skin lesions such as papules, pustules or retention skin lesions such as cysts or comedones, **characterized in that** the composition is prepared such as defined in any of claims 5 to 8.
